# EUROPEAN PATENT SPECIFICATION

(11) **EP 1 797 455 B1**
(45) Date of publication and mention of the grant of the patent: **08.07.2009**
(21) Application number: 05776023.3
(22) Date of filing: 01.08.2005
(51) Int. Cl.: G01S 15/89, G01S 15/58, A61B 8/06

(54) **ADJUSTABLE TRACING OF FLOW VELOCITIES IN DOPPLER VELOCITY SPECTRA**
EINSTELLBARE VERFOLGUNG VON STRÖMUNGSGESCHWINDIGKEITEN IN DOPPLER-GESCHWINDIGKEITS-SPEKTREN
TRACE REGLABLE DE VITESSES D'ECOULEMENT DANS DES SPECTRES DE VITESSE DOPPLER

(30) Priority: 30.08.2004 US 605636 P
(43) Date of publication of application: 20.06.2007
(73) Proprietor: Koninklijke Philips Electronics N.V., 5621 BA Eindhoven (NL)
(72) Inventor: MAN, Junzheng, Bothell, WA 98041-3003 (US); LU, Haiyuan, Bothell, WA 98041-3003 (US); ROBINSON, Marshall, Bothell, WA 98041-3003 (US); SAAD, Ashraf, Bothell, WA 98041-3003 (US); SKYBA, Dan, Bothell, WA 98041-3003 (US)
(74) Representative: van Oudheusden-Perset, Laure E.
(86) International application number: PCT/IB2005/052572
(87) International publication number: WO 2006/024975

(56) References cited:
- US-A- 5 634 465
- US-A- 6 050 948
- US-B1- 6 293 913
- PATENT ABSTRACTS OF JAPAN vol. 1997, no. 04, 30 April 1997 (1997-04-30) & JP 08 322841 A (MATSUSHITA ELECTRIC IND CO LTD), 10 December 1996 (1996-12-10)

## Description

This invention relates to medical diagnostic ultrasound systems and, in particular, to adjustable automated traces of spectral flow velocities.

US patents 5,287,753 and 5,634,465 illustrate automated techniques for tracing the mean and peak velocity levels in a spectral Doppler display. As each spectral line for the spectral Display is created, the ultrasound systems in these patents process the Doppler data to identify the peak and/or the mean velocity for each spectral line. In both patents this is done in consideration of the possible contamination of the Doppler data with noise, in the former patent with reference to external noise sources and in the latter patent with reference to system noise sources. When the mean and peak velocity levels are located in each spectral line as the line is produced, those points on the line can be identified visually as the spectral line is added to the scrolling spectral display and connected to the corresponding point or points on the previous spectral line. This enables the mean and/or peak velocity levels in the spectral display to be traced automatically in real time.

As the spectral display is produced it may be recorded and reviewed later for diagnosis or used for subsequent measurements or calculations of vascular performance. Usually the automated traces will seem correct to the clinician, but occasionally a trace may seem incorrectly located on the spectral display. When the clinician is confronted with an automated trace which seems to the clinician to be intuitively inaccurate the only option is for the clinician to manually trace what he or she feels are the correct values in the spectral display. Such manual retracing of, for example, the peak velocity values can be laborious and time consuming. However the clinician will generally resort to manual tracing in which he is confident, particularly when the traced levels are to be the basis for calculations of the patient's vascular performance. Accordingly it is desirable to provide some means for expediting the correction of an automated trace of a spectral display which seems incorrect to the user.

In accordance with the principles of the present invention, a diagnostic ultrasound system and method are described which enables a user to adjust an automated spectral display trace which seems inaccurate to the user. A user control is provided by which the user can select a point or points on the automated spectral trace and relocate the point or points to a desired location on the spectral display. As the point or points are manually relocated by the user the automated system automatically repositions the trace including, when necessary, repositioning adjoining points of the trace. In one embodiment this manual adjustment of the trace is facilitated by showing control points on the trace at evenly spaced locations or at local minima and/or maxima in the trace. In another embodiment the manual adjustment is facilitated by showing key physiological points on the trace which the user may adjust in time position, velocity location, or both. In another embodiment the manual adjustment is facilitated by a local manual redraw of the trace which can start anywhere on the trace and be re-connected to another point on the trace at an ending point on the trace, updating any key physiological points on the redrawn trace.

In the drawings:
FIGURE 1 illustrates in block diagram form an ultrasonic diagnostic imaging system constructed in accordance with the principles of the present invention;
FIGURE 2 illustrates a spectral Doppler display in which both the peak and mean velocity levels have been traced;
FIGURE 3 illustrates a preferred technique for tracing the peak and mean velocity levels in a high line density spectral display;
FIGURES 4a, 4b, and 4c illustrate a first embodiment of the present invention by which an automated spectral Doppler trace may be manually adjusted; and
FIGURES 5a, 5b, 5c and 5d illustrate a second embodiment of the present invention by which key points in a spectral Doppler trace are identified and adjusted.

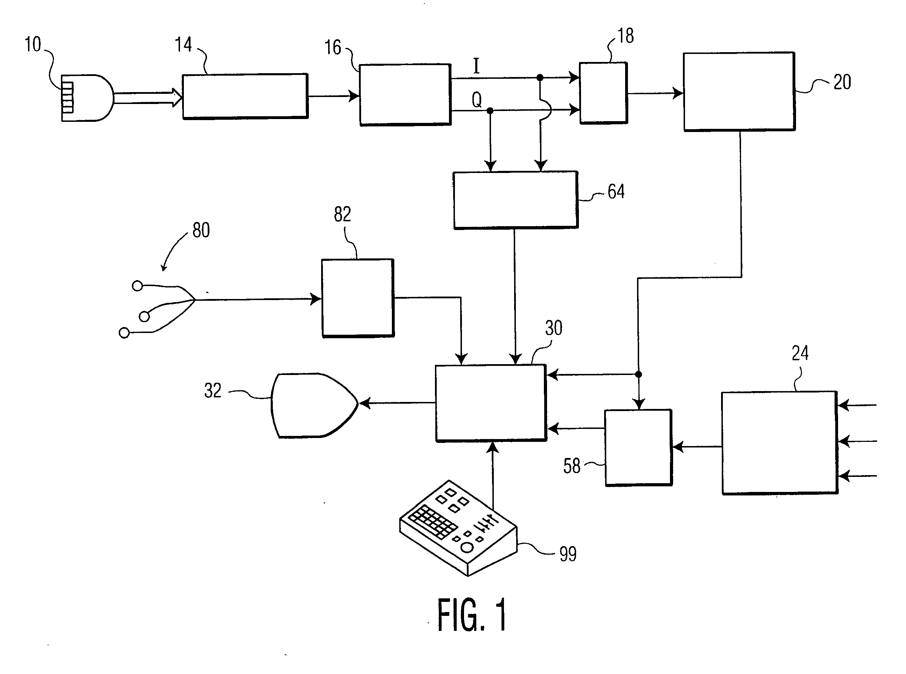

Referring first to FIGURE 1, an ultrasound system constructed in accordance with the principles of the present invention is shown in block diagram form. Ultrasonic signals are transmitted by the array transducer 10 of an ultrasound probe and resultant echoes are received by transducer elements. The received echo signals are formed into a single signal or beam by a beamformer 14. The echo signal information is detected by a Doppler detector 16 which produces quadrature I and Q signal components. A number of such signal components from the site in the body being diagnosed are applied to a Doppler processor 18, one form of which is a fast Fourier transform (FFT) processor, which computes the Doppler frequency shift of the received signals. This basic Doppler data is post-processed by a Doppler post processor 20, which further refines the data by techniques such as wall filtering, gain control, or amplitude compression.

Intermittently during the reception of Doppler echoes, B mode echoes are received. These echoes are also formed into I and Q components which may then be amplitude detected by taking the square root of the sum of the squares of the I and Q values in a B mode image processor 64. The B mode image processor also arranges the B mode echoes into a desired display form by scan conversion. The resultant two dimensional image is coupled to a Doppler display processor 30 where it may be displayed in a time interleaved manner with the spectral Doppler data.

The post processed Doppler data is applied to a peak velocity detector 58 and the Doppler display processor 30. The Doppler display processor uses the Doppler data for the display of a real time sequence of spectral line information. The peak velocity detector compares the Doppler data against a noise threshold NOISEₜₕ to determine the peak velocity point of a spectral line, as discussed more fully in US patents 5,287,753 and 5,634,465. The peak velocity detector 22 may also perform filtering of the Doppler data and may also be used to identify mean velocity levels as discussed more fully in the '753 patent. The Doppler display processor 30 then provides both an anatomical B mode image and a spectral Doppler display with peak and/or mean velocity values automatically traced as the discussed in the aforementioned patents.

The ultrasound display will also preferably show an ECG trace drawn in response to reception of an R-wave signal. The R-wave is the electrical physiological signal produced to stimulate the heart's contraction, and is conventionally detected by an electrocardiograph (ECG). FIGURE 1 shows a set of ECG electrodes 80 which may be affixed to the chest of a patient to detect the R-wave signal. The signal is detected and processed by an ECG signal processor 82 and applied to the Doppler display processor 30, which displays the ECG waveform in synchronism with the scrolling spectral Doppler display. The B mode image can be used to locate and display the point in the patient's anatomy at which the spectral information is acquired.

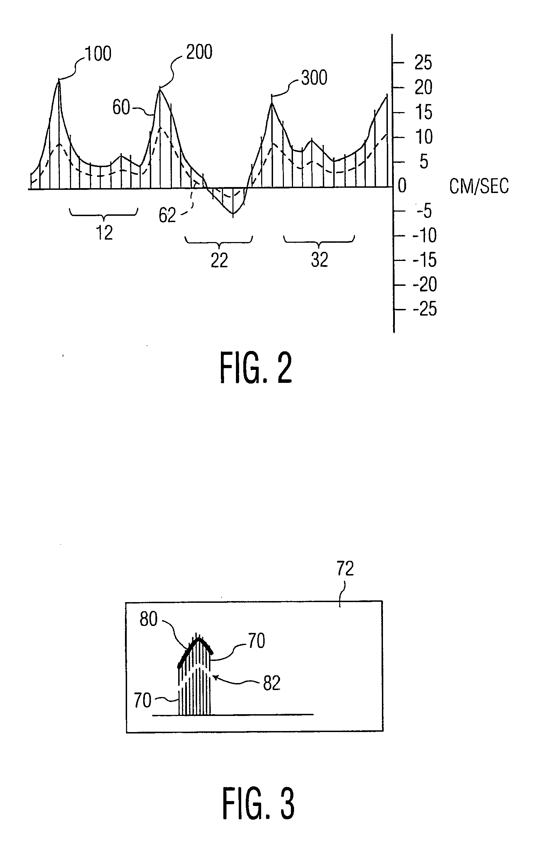

A typical spectral Doppler display as produced by an embodiment of the present invention is shown in FIGURE 2. Such a display generally comprises the Doppler information of discrete sampling periods displayed as a sequence of continuous scrolling spectral lines in a real time versus velocity display as shown in FIGURE 2. In the display of FIGURE 2, newly generated spectral lines are continually produced at the right side of the display. The sequence of lines moves or scrolls from right to left, with previously generated spectral data on the left and progressively more current data to the right. Each line conveys the range of flow velocities detected in the blood flow at a chosen location in the body at a particular time of Doppler interrogation. The highest velocities shown by lines 100, 200, and 300 would typically occur during the systolic phase of the heart cycle. The intervals 12, 22, and 32 between the systolic phases represent flow velocity during the intervening diastolic phases of heart action.

In accordance with the principles of the present invention, FIGURE 2 illustrates a spectral line display in which the peak velocity of each spectral (vertical) line has been identified and the peaks connected by the solid display line 60. As FIGURE 2 shows, the spectral line peak velocities can be identified and displayed as the spectral lines occur and are displayed, thereby providing a real time continuous display of traced peak spectral velocities. For each displayed spectral line which satisfies a noise immunity test a mean velocity value is also calculated and displayed. A variety of techniques are known for calculating mean velocity as discussed in the aforementioned '753 patent. The mean velocity is marked on the spectral line display, also concurrently with the initial appearance of the spectral line at the right-hand side of the spectral line display. FIGURE 2 shows a dashed line 62 which connects the calculated mean velocity values of the displayed spectral lines.

The peak and mean velocity values may be traced with separately distinguished lines as shown in FIGURE 2, or by differently colored lines. A preferred way to visually trace the peak and mean velocity values in a monochromatic high density spectral line display is shown in FIGURE 3. In this FIGURE the spectral lines 70 are displayed in shades of gray against a white background 72. The peak velocity line 80 is displayed as a sequence of black dots, each marking the peak velocity on its associated spectral line. The mean velocity values are marked by blanking the mean velocity positions on the respective spectral lines, thereby effectively leaving a white line running through the spectral lines 70 as indicated at 82. This technique takes advantage of rapid, high density production and display of spectral lines, in which the spectral lines 70 are displayed virtually adjacent to each other, thereby resembling a continuous band of gray shading below the peak velocity line 80. The white mean velocity line 82 is thus distinctly displayed in contrast to the surrounding gray shading of the spectral lines. One skilled in the art will realize that the display of FIGURE 3 is generally shown with black/white reversal in the typical ultrasound display.

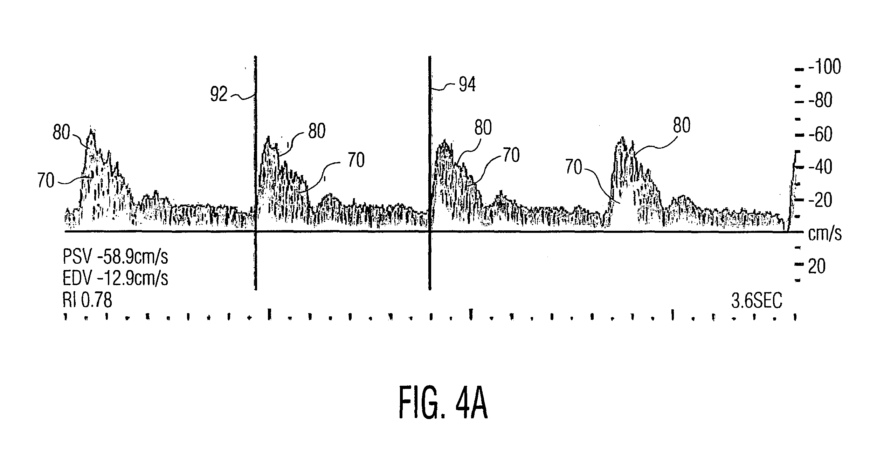

In accordance with the principles of the present invention, an automated tracing on a spectral display can be adjusted by the user as illustrated by FIGURES 4a-4c. In this first embodiment the peak velocities of spectral lines 70 in FIGURE 4a have been traced by the line 80, corresponding to the peak velocity display line 60 in FIGURE 2. The real time spectral display can be stopped (frozen) on the display screen by actuation of the "freeze" button on the ultrasound system control panel 99. Alternatively, a previously recorded real time spectral display can be replayed and frozen on the screen. In either case, the ultrasound system will automatically delineate the extent of the spectral lines of one heart cycle by vertical lines 92,94 known as "goalposts." The goalpost lines may be placed by examining the spectral waveform or trace for the end diastole minima. Alternatively, the goalpost lines may be located by relating the ECG trace to the spectral display when an ECG trace is available. The ultrasound system will then use the information of this heart cycle for calculations and measurements. If the user does not want to accept this heart cycle or prefers another, he may click on another heart cycle in the spectral display to reposition the goalpost lines 92,94, or drag the vertical goalpost lines with a screen cursor to frame a different heart cycle in the spectral display. The graphics on the lower left of the display show the numeric values of certain data points of the selected heart cycle and any calculations the user desires to see. In this example the graphics show the peak systolic velocity (PSV) value of -58.9 cm/sec, the end diastolic velocity (EDV) value of -12.9 cm/sec, and the resistivity index (RI) of 0.78.

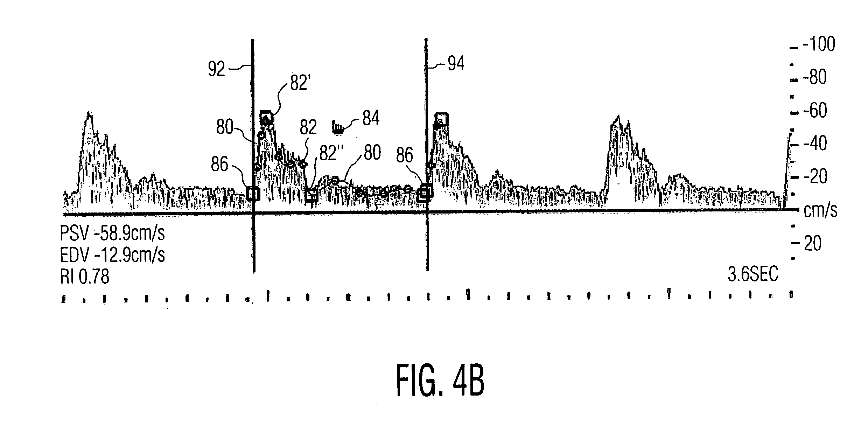

However, suppose that the user feels that the trace 80 has been incorrectly drawn. The user may doubt the calculated RI value, for instance, which may lead to the belief that the trace 80 is not accurately drawn. In such case, the user clicks on the "Edit Trace" menu item, which may be shown on the image display screen or on a touchscreen panel of the ultrasound system, or may be a separate control on the control panel 99. This selection will cause a series of control points 82,86 to appear on the trace 80 of the selected heart cycle, as shown in FIGURE 4b. In this embodiment the control points comprise a series of small markers 82 and larger markers 82', 82", and 86. The larger markers in this embodiment are located at key timing points of the heart cycle. In this case the marker 82' marks the peak systolic velocity point on the trace 80, the marker 82" marks the end systolic velocity point on the trace, and the marker 86 marks the end diastolic point on the trace. Also appearing on the screen is a cursor 84 which may be manipulated by a user control on the control panel 99 such as a trackball or mouse.

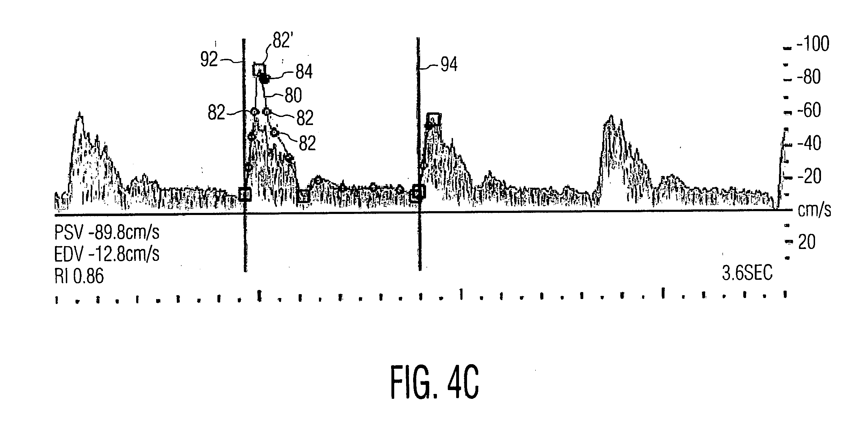

In this example the user feels that the peak systolic velocity point is actually higher than depicted by the automatically drawn trace 80. The user will then "grab" the control point 82' and "drag" it up to the desired velocity level as shown in FIGURE 4c. As the control point 82' is repositioned, the trace 80 and other control points 82 on the trace follow along with the repositioned control point 82'. This is done by recalculating the trace 80 on-the-fly by a spline interpolation technique, whereby the relocation of one point on the trace causes neighboring points on connecting spline curves to be automatically adjusted to provide a smooth trace. As the control points 82,82' and trace 80 are repositioned by the user, display values and calculations associated with the trace are also updated and recalculated on-the-fly. In this example it can be seen that the PSV value has been automatically updated to -89.8 cm/sec, the location of the repositioned control point 82' in FIGURE 4c, and the RI value has been affected by the adjustment and recalculated to 0.86. Thus, the user can visually see the adjustment he is making to the automatic trace 80 and can simultaneously see the effects of his adjustment on displayed and calculated values. These new display and calculated values can give the user confidence in the accuracy of his adjustments or lead to their further refinement by subsequent adjustment.

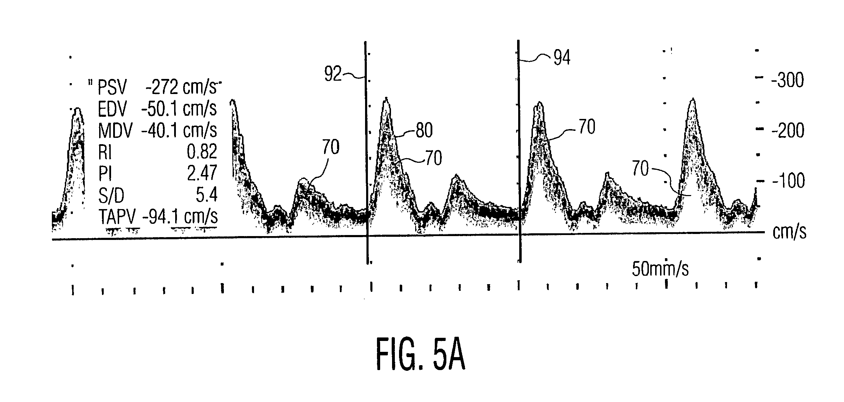

FIGURES 5a-5d illustrate a second embodiment of the present invention. In FIGURE 5a the lines 70 of a spectral display have their peak velocity values traced by a trace line 80 and a heart cycle is delineated by the goalpost lines 92,94. The numeric display shows another key point in the spectral display, the mean diastole velocity (MDV). Three other calculations are also displayed, the pulsatility index (PI), the systolic/diastolic ratio (S/D), and the time-averaged peak velocity (TAPV).

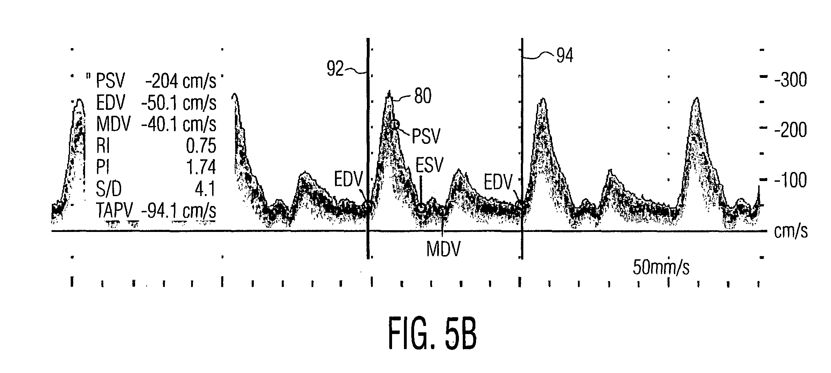

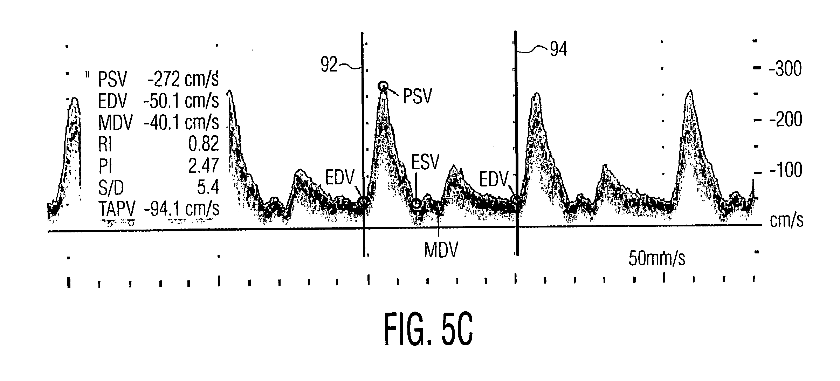

In FIGURE 5b the trace 80 has been supplemented with the addition of the display and identity of the key timing points of PSV, ESV, MDV, and EDV (end diastolic velocity). The key timing points to be displayed and identified can be chosen by the user and their location in time identified from the ECG waveform. The key timing points may also be calculated from the automatic tracing algorithm described in the aforementioned patents, which finds local maxima and minima as related to both the shape of the Doppler spectrum and the ECG waveform. The key timing points are displayed if the user chooses to show them (by turning them "on" via the control panel or user interface). The key timing points drive the results such as PSV, EDV and their derivative calculations. In this example it is seen that the PSV point is not located at the systolic peak of the tracing 80. In such case the user may reposition the point along the trace (*i.e*., in time) by grabbing the PSV point with the screen cursor and sliding the PSV marker to the systolic peak of the trace 80 as shown in FIGURE 5c. The graphics are updated correspondingly. It can be seen that the PSV value has increased from -204 cm/sec to -272 cm/sec in this example, and that the dependent RI, PI and S/D calculations have changed also.

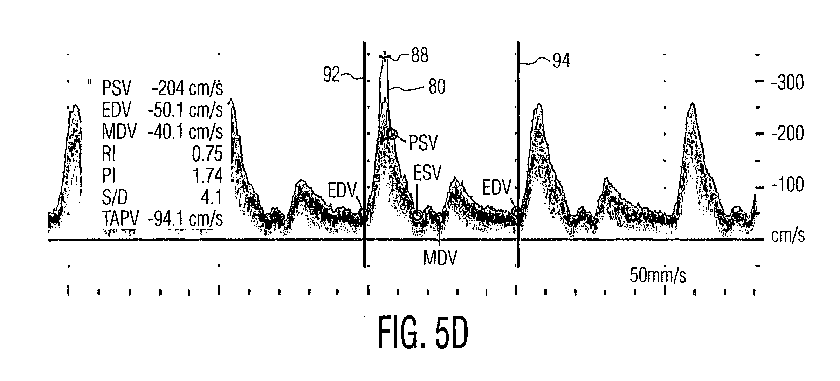

Alternatively or additionally, the user may feel that the trace 80 is incorrectly drawn. In such case the user may grab the trace 80 with a cursor 88 and drag the trace to the desired amplitude as shown in FIGURE 5d. As in the previous example, the trace 80 is recalculated and display on-the-fly, giving the appearance that the user is stretching the trace line to its new location. Alternatively, the user may click at one point on the trace and redraw a portion of the trace manually with a screen pointer until reconnecting with the trace at another point on the trace. In this example the user has redrawn the spectral peak on either side of the cursor 88. The newly recalculated graphic values at the left of the display show that this repositioning of the peak velocity trace has affected three of the four displayed calculations. In this embodiment there are no discrete control points for the user to grab. Instead, each point on the trace 80 may be grabbed and repositioned by the user's cursor to adjust the position of the automated spectral trace. Following the adjustment of the trace 80, key points are automatically adjusted to their optimal locations based on the tracing algorithm. However, if the automatic placement of the key points is deemed unsatisfactory, the user may reposition the key timing points on the trace manually. For instance, the PSV may be repositioned to the new systolic peak of the trace 80 in FIGURE 5d.

## Claims

1. An ultrasonic diagnostic imaging system for analyzing blood flow comprising:
means (10, 14) for acquiring spectral Doppler information;
a spectral Doppler analyze (16, 18, 20, 30, 58), responsive to the spectral Doppler information, which automatically traces at least one of the mean or peak velocity of a flow spectrum;
a display (32), coupled to the spectral Doppler analyzer, which displays the Doppler flow spectrum (70) with the tracer (80); and
a user control, operable with the display, by which the position of the trace relative to the Doppler flow spectrum may be manually adjusted.

2. The ultrasonic diagnostic imaging system of Claim 1, wherein the trace further includes a plurality of control points (82, 86) by which the position of the trace may be adjusted by operation of the user control.

3. The ultrasonic diagnostic imaging system of Claim 2, wherein the control points are uniformly distributed in time along the trace.

4. The ultrasonic diagnostic imaging system of Claim 2, wherein the control points are located at local minima and/or maxima of the trace.

5. The ultrasonic diagnostic imaging system of Claim 2, wherein the control points are located at key timing points of the heart cycle.

6. The ultrasonic diagnostic imaging system of Claim 5, wherein the control points are graphically identified on the display.

7. The ultrasonic diagnostic imaging system of Claim 5, wherein the Doppler flow spectrum has a velocity axis and a time axis; and
wherein the control points are adjustable in both the time and velocity dimensions.

8. The ultrasonic diagnostic imaging system of Claim 1, wherein the display further displays a trace adjustment cursor (84),
wherein the trace adjustment cursor is operable by the user control to adjust the position of the trace.

9. The ultrasonic diagnostic imaging system of Claim 8, wherein the trace adjustment cursor is operable by the user control to adjust the velocity position of the trace.

10. The ultrasonic diagnostic imaging system of Claim 8, wherein the trace further includes a plurality of control points; and
wherein the trace adjustment cursor is operable by the user control to adjust the position of a control point along the trace.

11. The ultrasonic diagnostic imaging system of Claim 10, wherein the control points define key timing points of the heart cycle.

12. The ultrasonic diagnostic imaging system of Claim 1, wherein the spectral Doppler analyzer further comprises means for delineating a heart cycle of a Doppler flow spectrum from which key values or calculations can be produced.

13. The ultrasonic diagnostic imaging system of Claim 12, wherein the display further comprises means for graphically displaying key values and/or calculations from a delineated heart cycle.

14. The ultrasonic diagnostic imaging system of Claim 13, wherein the spectral Doppler analyzer further comprises means for automatically updating a graphically displayed key value and/or calculation of a delineated heart cycle in response to adjustment of the position of the trace.

15. A method for adjusting an automatically drawn trace (80) of a spectral Doppler parameter comprising :
displaying a Doppler flow spectrum (70) on which a parameter has been traced (80);
grabbing a point on the trace with a display cursor (84) manipulated by a user control (99); and
dragging the point on the trace to a different position on the display (32).

16. The method of Claim 15, wherein grabbing further comprises grabbing a control point on the trace.

17. The method of Claim 16, wherein dragging further comprises moving the control point to a different position in time and/or velocity on the display.

18. The method of Claim 15, further comprising automatically fitting the trace on either side of the point to the different position of the point on the display.

19. The method of Claim 15, wherein grabbing further comprises grabbing a point on the trace defined as a key timing point of the heart cycle.

20. The method of Claim 15, further comprising displaying graphics of key value and/or calculation based upon the trace of a heart cycle,
wherein a graphically displayed key value and/or calculation is automatically updated in response to dragging a point on the trace to a different position.

## Patentansprüche

1. Diagnostisches Ultraschall-Bildgebungssystem zum Analysieren der Blutströmung, mit:
Mitteln (10, 14) zum Erfassen von spektralen Doppler-Informationen;
einem spektralen Doppler-Analysator (16, 18 20, 30, 58), der auf die spektralen Doppler-Informationen reagiert und automatisch mindestens entweder die mittlere Geschwindigkeit oder die Spitzengeschwindigkeit eines Strömungsspektrums verfolgt;
einem Display (32), das mit dem spektralen Doppler-Analysator verbunden ist und das Doppler-Strömungsspektrum (70) mit der Spur (80) anzeigt; und
einem Benutzerbedienelement, das mit dem Display zu betätigen ist und mit dessen Hilfe die Position der Spur relativ zum Doppler-Strömungsspektrum manuell angepasst werden kann.

2. Diagnostisches Ultraschall-Bildgebungssystem nach Anspruch 1, wobei die Spur weiterhin eine Vielzahl von Kontrollpunkten (82, 86) umfasst, mit denen die Position der Spur durch Betätigen des Benutzerbedienelements justiert werden kann.

3. Diagnostisches Ultraschall-Bildgebungssystem nach Anspruch 2, wobei die Kontrollpunkte zeitlich gleichmäßig entlang der Spur verteilt sind.

4. Diagnostisches Ultraschall-Bildgebungssystem nach Anspruch 2, wobei sich die Kontrollpunkte bei lokalen Minima und/oder Maxima der Spur befinden.

5. Diagnostisches Ultraschall-Bildgebungssystem nach Anspruch 2, wobei sich die Kontrollpunkte an wichtigen Timing-Punkten des Herzzyklus befinden.

6. Diagnostisches Ultraschall-Bildgebungssystem nach Anspruch 5, wobei die Kontrollpunkte auf dem Display grafisch **gekennzeichnet** sind.

7. Diagnostisches Ultraschall-Bildgebungssystem nach Anspruch 5, wobei das Doppler-Strömungsspektrum eine Geschwindigkeitsachse und eine Zeitachse hat; und
wobei die Kontrollpunkte sowohl in der Zeitdimension als auch in der Geschwindigkeitsdimension justierbar sind.

8. Diagnostisches Ultraschall-Bildgebungssystem nach Anspruch 1, wobei das Display weiterhin einen Spurjustierungscursor (84) anzeigt,
wobei der Spurjustierungscursor mit dem Benutzerbedienelement betätigt werden kann, um die Position der Spur zu justieren.

9. Diagnostisches Ultraschall-Bildgebungssystem nach Anspruch 8, wobei der Spurjustierungscursor mit dem Benutzerbedienelement betätigt werden kann, um die Geschwindigkeitsposition der Spur zu justieren.

10. Diagnostisches Ultraschall-Bildgebungssystem nach Anspruch 8, wobei die Spur weiterhin eine Vielzahl von Kontrollpunkten enthält; und
wobei der Spurjustierungscursor mit dem Benutzerbedienelement betätigt werden kann, um die Position eines Kontrollpunktes auf der Spur zu justieren.

11. Diagnostisches Ultraschall-Bildgebungssystem nach Anspruch 10, wobei die Kontrollpunkte wichtige Timingpunkte des Herzzyklus definieren.

12. Diagnostisches Ultraschall-Bildgebungssystem nach Anspruch 1, wobei der spektrale Doppler-Analysator weiterhin Mittel zum Abgrenzen eines Herzzyklus eines Doppler-Strömungsspektrums umfasst, anhand dessen wichtige Werte oder Berechnungen erzeugt werden können.

13. Diagnostisches Ultraschall-Bildgebungssystem nach Anspruch 12, wobei das Display weiterhin Mittel zum grafischen Anzeigen von wichtigen Werten und/oder Berechnungen aus einem abgegrenzten Herzzyklus umfasst.

14. Diagnostisches Ultraschall-Bildgebungssystem nach Anspruch 13, wobei der spektrale Doppler-Analysator weiterhin Mittel zum automatischen Aktualisieren eines grafisch angezeigten wichtigen Wertes und/oder einer derartigen Berechnung eines abgegrenzten Herzzyklus in Reaktion auf die Justierung der Spurposition umfasst.

15. Verfahren zum Justieren einer automatisch gezeichneten Spur (80) eines spektralen Doppler-Parameters, das Folgendes umfasst:
Anzeigen eines Doppler-Strömungsspektrums (70), auf dem ein Parameter verfolgt wurde (80);
Greifen eines Punktes auf der Spur mit einem Displaycursor (84), der durch ein Benutzerbedienelement (99) manipuliert wird; und
Ziehen des Punktes auf der Spur an einer andere Position auf dem Display (32).

16. Verfahren nach Anspruch 15, wobei das Greifen weiterhin das Greifen eines Kontrollpunktes auf der Spur umfasst.

17. Verfahren nach Anspruch 16, wobei das Ziehen weiterhin das Bewegen des Kontrollpunktes an eine andere Position bezüglich der Zeit und/oder Geschwindigkeit auf dem Display umfasst.

18. Verfahren nach Anspruch 15, weiterhin umfassend das automatische Anpassen der Spur auf beiden Seiten des Punktes an die andere Position des Punktes auf dem Display.

19. Verfahren nach Anspruch 15, wobei das Greifen weiterhin das Greifen eines Punktes auf der Spur umfasst, der als ein wichtiger Timing-Punkt des Herzzyklus definiert ist.

20. Verfahren nach Anspruch 15, weiterhin umfassend das Anzeigen der Grafik des wichtigen Wertes und/oder der Berechnung basierend auf der Spur eines Herzzyklus,
wobei ein grafisch angezeigter wichtiger Wert und/oder eine derartige Berechnung automatisch in Reaktion auf das Ziehen eines Punktes auf der Spur an eine andere Position aktualisiert werden.

## Revendications

1. Système d'imagerie de diagnostic ultrasonique pour une analyse d'écoulement sanguin comprenant :
des moyens (10, 14) pour acquérir des informations de Doppler spectral ;
un analyseur de Doppler spectral (16, 18, 20, 30, 58), répondant aux informations de Doppler spectral, qui trace automatiquement au moins l'une de la vitesse moyenne ou de la vitesse de pointe d'un spectre d'écoulement ;
un affichage (32), couplé à l'analyseur de Doppler spectral, qui affiche le spectre d'écoulement de Doppler (70) avec le tracé (80) ; et
une commande d'utilisateur, utilisable avec l'affichage, par laquelle la position du tracé par rapport au spectre d'écoulement de Doppler peut être manuellement ajustée.

2. Système d'imagerie de diagnostic ultrasonique selon la revendication 1, dans lequel le tracé comprend en outre une pluralité de points de commande (82, 86) par lesquels la position du tracé peut être ajustée en utilisant la commande d'utilisateur.

3. Système d'imagerie de diagnostic ultrasonique selon la revendication 2, dans lequel les points de commande sont uniformément répartis dans le temps le long du tracé.

4. Système d'imagerie de diagnostic ultrasonique selon la revendication 2, dans lequel les points de commande sont situés à des minima et/ou maxima locaux du tracé.

5. Système d'imagerie de diagnostic ultrasonique selon la revendication 2, dans lequel les points de commande sont situés à des points de timing essentiels du cycle cardiaque.

6. Système d'imagerie de diagnostic ultrasonique selon la revendication 5, dans lequel les points de commande sont identifiés graphiquement sur l'affichage.

7. Système d'imagerie de diagnostic ultrasonique selon la revendication 5, dans lequel le spectre d'écoulement de Doppler a un axe de vitesse et un axe de temps ; et
dans lequel les points de commande sont ajustables dans les dimensions de temps et de vitesse.

8. Système d'imagerie de diagnostic ultrasonique selon la revendication 1, dans lequel l'affichage affiche en outre un curseur d'ajustement de tracé (84),
dans lequel le curseur d'ajustement de tracé est utilisable par la commande d'utilisateur pour ajuster la position du tracé.

9. Système d'imagerie de diagnostic ultrasonique selon la revendication 8, dans lequel le curseur d'ajustement de tracé est utilisable par la commande d'utilisateur pour ajuster la position de vitesse du tracé.

10. Système d'imagerie de diagnostic ultrasonique selon la revendication 8, dans lequel le tracé comprend en outre une pluralité de points de commande ; et
dans lequel le curseur d'ajustement de tracé est utilisable par la commande d'utilisateur pour ajuster la position d'un point de commande le long du tracé.

11. Système d'imagerie de diagnostic ultrasonique selon la revendication 10, dans lequel les points de commande définissent des points de timing essentiels du cycle cardiaque.

12. Système d'imagerie de diagnostic ultrasonique selon la revendication 1, dans lequel l'analyseur de Doppler spectral comprend en outre des moyens de délinéation d'un cycle cardiaque d'un spectre d'écoulement de Doppler duquel des valeurs essentielles ou des calculs peuvent être produits.

13. Système d'imagerie de diagnostic ultrasonique selon la revendication 12, dans lequel l'affichage comprend en outre des moyens pour afficher graphiquement des valeurs essentielles et/ou des calculs d'un cycle cardiaque délinéé.

14. Système d'imagerie de diagnostic ultrasonique selon la revendication 13, dans lequel l'analyseur de Doppler spectral comprend en outre des moyens pour mettre automatiquement à jour une valeur essentielle et/ou un calcul affiché graphiquement d'un cycle cardiaque délinéé en réponse à l'ajustement de la position du tracé.

15. Procédé d'ajustement d'un tracé réalisé automatiquement (80) d'un paramètre de Doppler spectral comprenant les étapes consistant à :
afficher un spectre d'écoulement de Doppler (70) sur lequel un paramètre a été tracé (80) ;
prendre un point sur le tracé avec un curseur d'affichage (84) manipulé par une commande d'utilisateur (99) ; et
déplacer le point sur le tracé à une position différente sur l'affichage (32).

16. Procédé selon la revendication 15, dans lequel l'étape de prise comprend en outre l'étape consistant à prendre un point de commande sur le tracé.

17. Procédé selon la revendication 17, dans lequel l'étape de déplacement comprend en outre l'étape consistant à déplacer le point de commande à une position différente dans le temps et/ou en vitesse sur l'affichage.

18. Procédé selon la revendication 15, comprenant en outre l'étape consistant à adapter automatiquement le tracé d'un côté ou de l'autre du point à la position différente du point sur l'affichage.

19. Procédé selon la revendication 15, dans lequel l'étape de prise comprend en outre l'étape consistant à prendre un point sur le tracé défini en tant que point de timing essentiel du cycle cardiaque.

20. Procédé selon la revendication 15, comprenant en outre l'affichage de graphiques de la valeur essentielle et/ou du calcul sur la base du tracé d'un cycle cardiaque,
dans lequel une valeur essentielle et/ou un calcul affiché graphiquement est automatiquement mis à jour en réponse au déplacement d'un point sur le tracé à une position différente.
